Europäisches Patentamt

(19) European Patent Office

Office européen des brevets

(11) Publication number: **0 067 680**
**B1**

(12) **EUROPEAN PATENT SPECIFICATION**

(45) Date of publication of patent specification: **28.11.84**

(21) Application number: **82303023.4**

(22) Date of filing: **11.06.82**

(51) Int. Cl.³: **A 61 B 17/38**

(54) **Electrocautery needle and method of forming it.**

(30) Priority: **12.06.81 US 272853**

(43) Date of publication of application:
**22.12.82 Bulletin 82/51**

(45) Publication of the grant of the patent:
**28.11.84 Bulletin 84/48**

(84) Designated Contracting States:
**AT BE CH DE FR IT LI NL SE**

(56) References cited:
**FR-A-2 252 191**
**US-A-3 682 162**
**US-A-4 178 067**

(73) Proprietor: **RAYCHEM CORPORATION**
**300 Constitution Drive**
**Menlo Park California 94025 (US)**

(72) Inventor: **Bloom, William George**
**10429 Reseda Boulevard**
**Northridge California 91326 (US)**
Inventor: **Geschwind, Gary Ira**
**700 Ames Avenue**
**Palo Alto California 94303 (US)**
Inventor: **Jervis, James Elliot**
**495 Walsh Road**
**Atherton California 94025 (US)**

(74) Representative: **Dlugosz, Anthony Charles et al**
**Raychem Limited Intellectual Property Law**
**Department Faraday Road**
**Dorcan Swindon Wiltshire (GB)**

Courier Press, Leamington Spa, England.

## Description

This invention relates to electrocautery needles and methods of forming them.

Bi-polar electrocautery tools have been used for a variety of electrosurgical procedures. For example, where a very controlled, localized current concentration is required, such as neuro- and eye surgery the bi-polar needle is especially useful. Currently, for these procedures, a forcep type probe is used. Each leg of the forcep is electrically isolated and insulated up to the extreme end of the tip. Only when the ends of the tip are brought into close proximity is there current flow, and then only across the gap between the tips. While this structure permits extremely accurate surgical procedures involving only minute amounts of tissue, the area of tissue burn must first be exposed to be reached with the forceps. Typically, a substantial amount of surgical cutting is required to locate the correct site for such forcep type electro-needles. Further, typical needles are expensive and not disposable, which leads to a variety of maintenance problems, including cleaning, resterilization, and maintenance of extremely delicate surgical instruments.

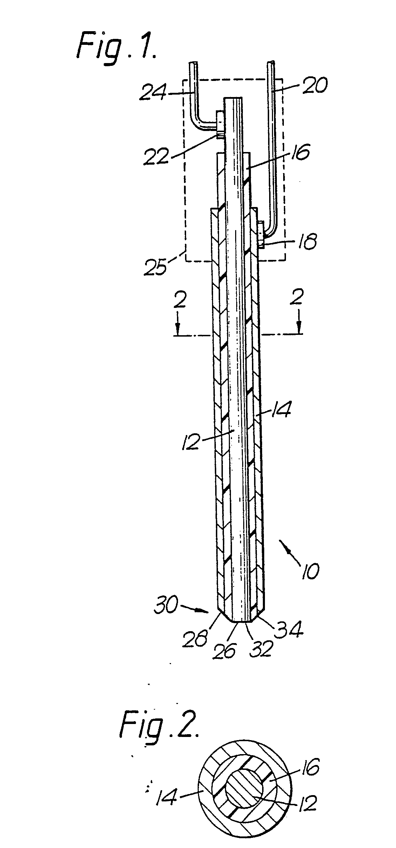

One form of bi-polar needle includes an inner and outer electrode and an intermediate layer of epoxy resin. The epoxy layer tends to break down upon the above described maintenance. These bi-polar tools are exmplified by Colyer, U.S. Letters Patent 3,682,162, which includes a small diameter hypodermic needle inserted into a larger diameter hypodermic needle and bonded together with an epoxy resin or other thermosetting plastics material as an intermediate layer. The history of such bi-polar needles is summarized in Cosens et al., U.S. Letters Patent 4,034,762, which is incorporated herein by reference. Cosens et al. recognize the problem of such bi-polar needles, and describe a needle which can be re-usable and withstand the rigours of maintenance. For instance, as seen in Figures 1 and 2 of Cosens et al., a cap is required to hold the electrodes together while dielectric tubing is used to insulate the electrodes. However, even Cosens et al. uses epoxy to secure the outer electrode to the cap.

The present invention provides an electrocautery needle, which comprises an elongate inner electrode located within and extending along a hollow elongate outer electrode, and a hollow insulating element disposed between the electrodes, the insulating element having been stretched in the axial direction from an intial configuration in which its external diameter is at least as great as the internal diameter of the outer electrode to a second configuration of smaller external diameter, and having been allowed to recover toward its initial configuration after it and the inner electrode have been inserted within the outer electrode so that it recovers against the electrodes and maintains them in their correct relative position.

When the insulating element is stretched in the axial direction its wall thickness, and hence its external diameter, will decrease, thereby allowing the hollow outer electrode to be slipped over the inner electrode and insulating element. When the insulating element is caused to recover it will contract axially and its wall thickness will increase so that the insulating element will be in compression in the radial direction and will maintain the two electrodes in their correct position, preferably so that the inner electrode is held substantially coaxially within the outer electrode.

The insulating element may, if desired, be formed from an elastomeric material so that it can be inserted into the outer electrode while being held in axial tension and will recover to its intial configuration as soon as the axial tension is released. Preferably, however, the insulating element is dimensionally recoverable so that it will remain in its stretched configuration until the appropriate treatment, e.g. application of solvent for a solvent-recoverable material. Most preferably the insulating element is dimensionally heat-recoverable. Heat recoverable articles are articles the dimensional configuration of which may be made substantially to change when subjected to heat treatment.

Usually these articles recover, on heating, towards an original shape from which they have previously been deformed but the term 'heat-recoverable", as used herein, also includes an article which, on heating, adopts a new configuration, even if it has not been previously deformed.

In thier most common form, such articles comprise a heat-shrinkable sleeve made from a polymeric material exhibiting the property of elastic or plastic memory as described, for example, in U.S. Patents 2,027,962; 3,086,242 and 3,957,372. As is made clear in, for example, U.S. Patent 2,027,962, the original dimensionally heat-stable form may be a transient form in a continuous process in which, for example, an extruded tube is expanded, whilst hot, to a dimensionally heat-unstable form but, in other applications, a preformed dimensionally heat stable article is deformed to a dimensionally heat-unstable form in a separate stage.

In the production of heat recoverable articles, the polymeric material may be cross-linked at any stage in the production of the article that will enhance the desired dimensionally recoverability. One manner of producing a heat-recoverable article comprises shaping the polymeric material into the desired heat-stable form, subsequently cross-linking the polymeric material, heating the article to a temperature above the crystalline melting point or, for amorphous materials the softening point, as the case may be, of the polymer, deforming the article and cooling the article whilst in the deformed state so that the deformed state of the article is retained. In use, since the deformed state of the article is heat-unstable, application of heat will cause the article to assume its original heat-stable shape.

In other articles, as described, for example, in British Patent 1,440,524, an elastomeric member such as an outer tubular member is held in a stretched state by a second member, such as an inner tubular member, which, upon heating weakens and thus allows the elastomeric member to recover.

The electrocautery needle according to the present invention has the advantage that it enables the needle to incorporate an insulating layer that can be cleaned and resterilized if desired without breakdown of the insulation. In addition, in view of the simple method of forming the needle, it is possible for the needle to be formed sufficiently inexpensively that the needle can be disposable after use.

The needle also has the advantage that one end of the electrodes may be shaped to form a variety of configurations, for example by grinding.

Thus, if the inner electrode is solid, the end of the electrodes may be ground to a point, preferably having a conical shape. Alternatively the inner electrode may itself be hollow, for example in the form of a cannula, if the needle is intended to perform irrigation or suction operations, in which case the end of the electrodes may be ground to a tip having a substantially frusto-conical profile.

If the needle is intended to perform both irrigation and suction operations it is preferable for the inner electrode to have a plurality of separate channels extending through it, for example one channel for introduction of irrigating fluid and another channel for removal of the fluid by suction. The use of a needle having an inner electrode with only a single channel for both irrigation and suction is also possible if an appropriate suction/irrigation valve mechanism is used.

Preferably the insulating element has a configuration and/or is formed from a material such that it can withstand a high power consumption of the needle, preferably at least 10 watts and especially at least 100 watts, without any electrical breakdown. Materials from which the insulating element is preferably formed include fluoropoylmers and especially a block copolymer comprising tetrafluoroethylene units and ethylene units sold by E. I. duPont de Nemours & Co. under the trade name "Tefzel".

The electrocautery needle according to the present invention may be formed by

(a) inserting with a hollow elongate outer electrode a hollow insulating element that has an elongate inner electrode located within it, the insulating element having an initial configuration in which its external diameter is at least as great as the internal diameter of the outer electrode and being in an axially stretched configuration in which its external diameter is less than the internal diameter of the outer electrode, and

(b) causing the insulating element to recover toward its unstretched configuration so that it recovers against the electrodes and maintains them in their correct relative position.

The insulating element may be positioned about the inner electrode during manufacture of the needle, and then stretched in its axial direction before insertion into the outer electrode, or the insulating element and the inner electrode may together constitute part of an electrical wire, the insulation or jacket of the wire forming the insulating element and the wire conductor forming the inner electrode. Where the insulating element and the inner electrode constitute part of an electrical wire, the insulation or jacket of the wire may be stretched axially either during formation of the needle or during manufacture of the wire.

The electrocautery needle may easily be connected to a power source and may be used in either a bipolar mode or a monopolar mode. In the bipolar mode electrical current flows between the two electrodes at the tip. In the monpolar mode only one of the electrodes, preferably the inner electrode is connected to the power source and the other terminal of the power source, preferably at earth potential, is connected to the patient *via* a grounding pad so that the needle can be used for electrical cauterization or other forms of surgical diathermy by fulguration.

Several forms of needle in accordance with the invention will now be described by way of example with reference to the accompanying drawings, in which:

Figure 1 is a longitudinal crossectional view of an electrocautery needle in accordance with this invention;

Figure 2 is a cross-sectional view of the electrocautery needle taken along line 2—2 of Figure 1;

Figures 3A, B, C, and D are schematic illustrations of the steps of forming the electrocautery needle in accordance with this invention;

Figures 4A, B, and C are schematic illustrations of the steps of an alternative method of making the electrocautery needle in accordance with this invention; and

Figures 5A and B are transverse cross-sectional views through further forms of electrocautery needle in accrodance with this invention.

Referring to the drawings, wherein like reference characters designate like or corresponding parts throughout the several views, and referring intiailly to Figures 1 and 2, there is shown bi-polar electrocautery needle 10 which includes an inner electrode 12 which may be either hollow or solid depending upon user needs. The bi-polar electrocautery needle also includes an outer electrode 14 which as seen in Figure 2 is concentric with the inner electrode 12. Between the electrodes 12 and 14 is located an insulating element 16.

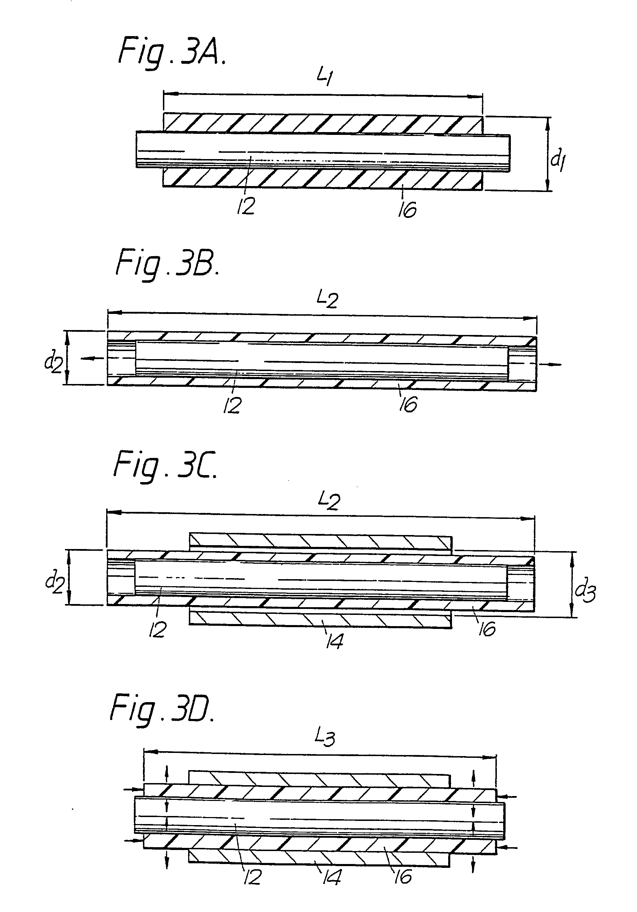

The needle may be formed by the process shown schematically in Figures 3A to D in which the insulating element 16 is initially positioned about the inner electrode 12 as shown in Figure 3A.

The insulating element 16 may be elastic, meaning that when stretched from its normal condition having length $1_1$ and diameter $d_1$, it tends to recover thereto. The element 16 may instead be heat-recoverable, so that when stretched, the element may be recoverable to its original configuration by heating. As will be appreciated, when stretching such an element axially, it also deforms radially, reducing its outside diameter. The outside diameter of the element 16 is so chosen that it is larger than the inside diameter $d_3$ of the outer electrode 14 so, when the element 16 in the unstretched condition, the outer electrode 14 could not be slipped over the inner electrode 12 with attached element 16.

As shown in Figure 3B, the insulating element 16 is then stretched in the direction of the arrows to length $1_2$, thereby radially reducing the element to outer diameter $d_2$ which is less than the inner diameter $d_3$ of the outer electrode. As shown in Figure 3C the outer electrode 14 is then slipped over the inner electrode 12 and stretched element 16. After correctly positioning the electrodes 12 and 14 relative to one another, the element 16 is recovered or relaxed toward its initial, unstretched configuration as shown in Figure 3C. Since the element 16 is elastic or heat-recoverable, it attempts to return to its normal diameter $d_1$ and length $1_1$, but, because the outside diameter $d_1$ of the element 16 is greater than the inside diameter $d_3$ of the outer electrode 14, the normal dimensional conditions ($1_1$ and $d_1$) of the element 16 will not be fully realized. Instead, since the element 16 is in radial compression, it wil continuously exert a radial force against the electrodes 12 and 14 in the direction of the arrows of Figure 3D, thereby maintaining the electrode in the correct relative position to one another.

The element 16 is made from a material having the desired dielectric constant if the needle is intended to be used with high frequency current. Among the materials which may be used are fluoropolymers especially that sold by E. I. duPont de Nemours & Co. under the trade name "Tefzel".

The element 16 is preferably formed from a heat-recoverable, cross-linked polymeric material so that it may withstand high power carried by the electrodes. As is well-known, cross-linked materials do not melt at temperatures above their transition states (see, e.g. U. S. Letters Patent 3,721,749). When the powers input of the needle is high, for example greater than 50 watts, the temperature becomes quite hot, and would usually melt thermoplastic materials. Cross-linked polymeric material do not suffer from such problems and therefore, are quite useful when high power (greater than 50 watts) is used.

In previous bi-polar needles, for example, Colyer, U.S. Letters Patent 3,682,162, the intermediate layer breaks down and fails to perform its insulating function when high power (greater than 10 watts) is carried by the electrodes. The Colyer structure is specifically limited to a bi-polar needle carrying 100 milliamps at a voltage of between 1 and 10 Volts or a maximum of 1 watt. In contrast, the structure according to the present invention is capable of withstanding 100 or more watts.

In the preferred embodiment, the recovered length of the means $1_3$ is less than the length of the inner electrode, and the length of the outer electrode is less than $1_3$. This enables the assembled bi-polar needle easily to be connected to a power source. For example, as illustrated in Figure 1, electrodes 12 and 14 may be easily connected to power source at one end, whilst the other end may be ground into a tip.

The electrodes 12 and 14 may be joined to leads as shown in Figure 1. A connector 18 connects electrode 14 to an electrical lead 20, and a connector 22 connects electrode 12 to another electrical lead 24. The needle may then be inserted into a handle or directly tied to a power source. In use, the handle portion 25 insulates the electrical current carried by the electrode so as not to cause shock or other uncomfortable side effects.

In the ordinary course, the user will install the needle 10 into a handle 25, which would be tied to a power source. Since the bi-polar needle 10 is inexpensive, it may be used and disposed of without the need for cleaning.

The electrodes are terminated at their ends 26 and 28 in the form of a tip 30. Using this system, it is possible easily to form the type of tip desired. In configuration shown, the tip 30 may be formed in a generally frusto-conical configuration having a flat section 32 with a bevelled side 34. It will be appreciated that where the inner electrode 12 is a solid electrode, it may be desirable to sharpen the tip 30 to a pencil point-like structure (not shown) where the needle is intended to be used in the fulguration mode.

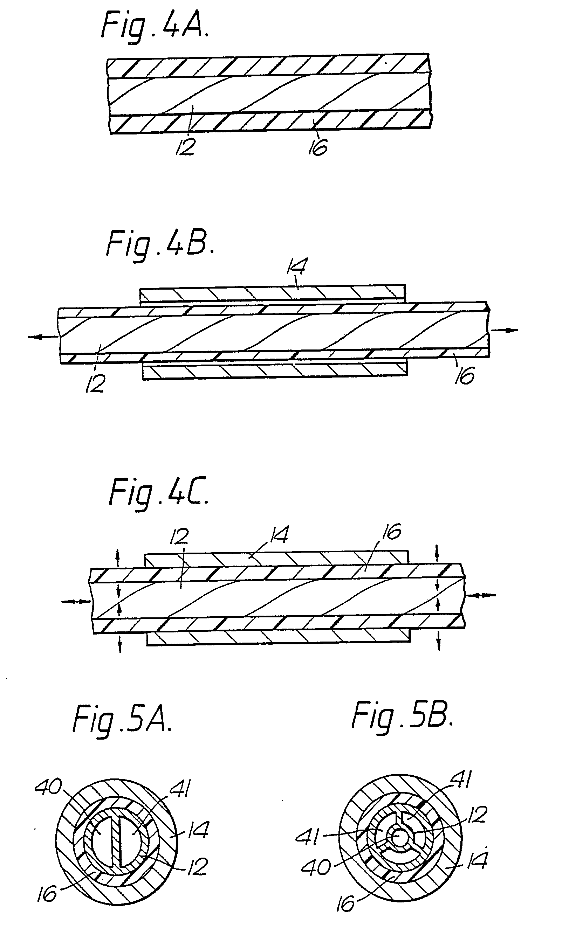

Figures 4A to C shown an alternative method of forming a bi-polar electrocautery needle which is less expensive than the method shown in Figures 3A to D. In this case, use is made of a pre-made insualted wire. The wire includes an inner core 12 and a dielectric jacket 16 which is formed from a recoverable material and constitutes the insulating element. The entire wire may be expanded, but preferably only the outer material, to an axially stretched, radially reduced condition. As shown in Figure 4B, an outer electrode 14 may then be slipped over the stretched wire and the jacket 16 may then be recovered in the fashion described above to form the structure shown in Figure 4C. Thus, for manufacturing ease, it has been found that it is desirable to form the inner electrode and insulating element by selection of a particular diameter wire already having a jacket. Preferably the jacket 16 is pre-expanded before or during extrusion over the core 12. The preferred wire for forming the inner electrode and insulating element is that sold by Raychem Corporation of Menlo Park, California, U.S.A. under the trade name "55 Wire" having a jacket formed from a fluoropolymer sold by E. I. duPont de Nemours & Co. under the trade name "Tefzel".

Figures 5A and B are transverse cross-sections through further forms of needle in which the inner electrode 12 has a plurality of channels extending through it. As shown in Figure 5A, channel 40 can be used for supply of irrigating fluid whilst channel 41 can be used for suction. As shown in Figure 5B, the inner electrode is divded into a central channel 40 which may, for example be used for suction and a number of other channels which may be used to dispense one or more different fluids.

## Claims

1. An electrocautery needle (10), which comprises an elongate inner electrode (12), located within and extending along a hollow elongate outer electrode (14), and a hollow insulating element (16) disposed between the electrodes, the insulating element having been stretched in the axial direction from an initial configuration in which its external diameter is at least as great as the internal diameter of the outer electrode to a second configuration of smaller external diameter, and having been allowed to recover toward its initial configuration after it and the inner electrode have been inserted within the outer electrode so that it recovers against the electrodes and maintains them in their correct relative position.

2. A needle as claimed in claim 1, wherein the insulating element comprises a dimensionally heat-recoverable polymeric material that has been heated to cause it to recover toward its initial configuration.

3. A needle as claimed in claim 1 or claim 2, wherein one end of the electrodes has been shaped by grinding.

4. A needle as claimed in any one of claims 1 to 3, wherein the inner electrode is hollow.

5. A needle as claimed in claim 4, wherein the inner electrode has a plurality of separate channels (40, 41) extending through it.

6. A needle as claimed in any one of claims 1 to 5, wherein the insulating element comprises a fluoropolymer.

7. A needle as claimed in claim 6, wherein the insulating element comprises a block copolymer comprising tetrafluoroethylene units and ethylene units.

8. A method of forming an electrocautery needle, which comprises:

(a) inserting within a hollow elongate outer electrode (14) a hollow insulating element (16) that has an elongate inner electrode (12) located within it, the insulating element having an initial configuration in which its external diameter is at least as great as the internal diameter of the outer electrode and being in an axially stretched configuration in which its internal diameter is less than the internal diameter of the outer electrode, and

(b) causing the insulating element to recover toward its unstretched configuration so that it recovers against the electrodes and maintains them in their correct relative position.

9. A method as claimed in claim 8, wherein the hollow insulating element constitutes the insulation or jacket of an electrical wire and the inner elongate inner electrode constitutes the conductor of the electrical wire.

10. A method as claimed in claim 9, wherein the insulating element has been axially stretched during manufacture of the electrical wire.

11. A method as claimed in claim 8 or claim 9, which includes the step of stretching the insulating element in the axial direction before the insulating element and inner electrode are inserted within the outer electrode.

12. A method as claimed in claim 8, which includes the steps of

(i) positioning the insulating element about the inner electrode, and then

(ii) stretching the insulating element in the axial direction to reduce its external diameter before it and the inner electrode are positioned within the outer electrode.

13. A method as claimed in any one of claims 8 to 12, wherein the insulating element is dimensionally heat-recoverable and is caused to recover by heating it.

## Patentansprüche

1. Elektrokauternadel (10), welche umfaßt: innerhalb einer hohlen Außenelektrode (14) und in deren Längsrichtung ver-laufende langgestreckte Innenelektrode (12) sowie ein hohles Isolierelement (16) zwischen den Elektroden, wobei das Isolierelement in axialer Richtung von einer Anfangsform, bei welcher sein Außendurchmesser mindestens ebenso groß ist wie der Innendurchmesser der Außenelektrode, zu einer zweiten Form mit geringerem Außendurchmesser gedehnt worden ist und wobei ihm die Möglichkeit gegeben worden ist, in seine Anfangsform zurückzukehren, nachdem es und die Innenelektrode in die Außenelektrode hineingesetzt worden sind, so daß es sich gegen die Elektroden zurückstellt und diese in ihrer richtigen gegenseitigen Anordnung festhält.

2. Nadel nach Anspruch 1, worin das Isolierelement ein in seinen Abmessungen wärmerückstellbares Polymermaterial unfaßt, das erwärmt wurde, um es zu veranlassen, zu seiner Anfangsform zurückzukehren.

3. Nadel nach Anspruch 1 oder Anspruch 2, worin das eine Ende der Elektroden durch Schliefen geformt ist.

4. Nadel nach einem der Ansprüche 1 bis 3, worin die Innenelektrode hohl ausgeführt ist.

5. Nadel nach Anspruch 4, worin die Innenelektrode eine Mehrzahl von getrennten Kanälen (40, 41) aufweist, die durch die Elektrode hindurchführen.

6. Nadel nach einem der Amsprüche 1 bis 5, worin das Isolierelement ein Fluorpolymer enthält.

7. Nadel nach Anspruch 6, worin das Isolierelement ein Blockcopolymeres umfaßt, das Tetrafluorethylen- und Ethyleneinheiten enthält.

8. Verfahren zum Herstellen einer Elektrokauternadel, welches umfaßt:

(a) Einführen eines hohlen Isolierelements (16), in dem sich eine langgestreckte Innenelektrode (12) befindet, in eine hohle, langgestreckte Außenelektrode (14), wobei das Isolierelement eine Anfangsform aufweist, bei der sein Außendurchmesser mindestens so groß ist wie der Innendurchmesser der Außenelektrode, und wobei es sich in einer axial gedehnten Form befindet, bei der sein Außendurchmesser kleiner ist als der Innendurchmesser der Außenelektrode, und.

(b) das Isolierelement veranlassen, sich in Richtung auf seine nichtgedehnte Form rückzustellen, so daß es sich gegen die Elektroden zurückstellt und diese in ihrer richtigen gegenseitigen Anordnung hält.

9. Verfahren nach Anspruch 8, worin das hohle Isolierelement die Isolierung oder Ummantelung eines elektrischen Drahts darstellt und die innenliegende langgestreckte Innenelektrode den Leiter des elektrischen Drahtes bildet.

10. Verfahren nach Anspruch 9, worin das Isolierelement während der Herstellung des elektrischen Drahtes in axialer Richtung gedehnt worden ist.

11. Verfahren nach Anspruch 8, oder Anspruch 9, das den Schritt des Dehnens des Isolierelements in axialer Richtung vor dem Einsetzen des Isolierelements und der Innenelektrode in die Außenelektrode aufweist.

12. Verfahren nach Anspruch 8, das folgende Schritte umfaßt:

(I) das Isolierelement um die Innenelektrode legen, und anschließend

(II) das Isolierelement in axialer Richtung dehnen, um dessen Außendurchmesser zu verringern, bevor es und die Innenelektrode in die Außenelektrode eingesetzt werden.

13. Verfahren nach einem der Ansprüche 8 bis 12, worin das Isolierelement bezüglich seiner Abmessungen wärmerückstellbar ist und durch Erwärmen zum Rückstellen veranlaßt wird.

**Revendications**

1. Une aiguille d'électrocautérisation (10) qui comprend une électrode intérieure allongée (12) disposée à l'intérieur d'une électrode extérieure allongée creuse (14) dans le sens longitudinal de laquelle elle s'étend et un élément isolant creux (16) disposé entre les électrodes, l'élément isolant ayant été étiré dans la direction axiale hors d'une configuration initiale dans laquelle son diamètre extérieur est au moins aussi grand que le diamètre intérieur de l'électrode extérieure et mis sous une seconde configuration de plus petit diamètre extérieur et ayant pu revenir vers sa configuration initiale après que cet élément et l'électrode intérieure ont été insérés à l'intérieur de l'électrode extérieure de sorte qu'il reprend sa forme contre les électrodes et les maintient dans leur position relative correcte.

2. Une aiguille telle que revendiquée dans la revendication 1, dans laquelle l'élément isolant est en une matière polymère à reprise thermique de ses dimensions que l'on a chauffée pour provoquer sa reprise vers sa configuration initiale.

3. Une aiguille telle que revendiquée dans la revendication 1 ou la revendication 2, dans laquelle une extrémité des électrodes a été façonnée par meulage.

4. Une aiguille telle que revendiquée dans l'une quelconque des revendications 1 à 3, dans laquelle l'électrode intérieure est creuse.

5. Une aiguille telle que revendiquée dans la revendication 4, dans laquelle l'électrode intérieure est traversée par plusieurs canaux séparés (40, 41).

6. Une aiguille telle que revendiquée dans l'une quelconque des revendications 1 à 5, dans laquelle l'élément isolant est fabriqué en un fluoropolymère.

7. Une aiguille telle que revendiquée dans la revendication 6, dans laquelle l'élément isolant est fabriqué en un polymère séquencé qui comprend des unités tétrafluoroéthylène et des unités éthylène.

8. Un procédé de fabrication d'une aiguille d'électrocautérisation, qui consiste:

(a) à introduire, dans une électrode extérieure allongée creuse (14), un élément isolant creux (16) à l'intérieur duquel est disposée une électrode intérieure allongée (12), l'élément isolant ayant une configuration initiale dans laquelle son diamètre extérieure est au moins aussi grand que le diamètre intérieur de l'électrode extérieure et étant dans une configuration axialement étirée dans laquelle son diamètre extérieur est inférieur au diamètre intérieur de l'électrode extérieure; et

(b) à provoquer le retour de l'élément isolant vers sa configuration non étirée de sorte qu'il reprend sa forme contre les électrodes et les maintient dans leur position relative correcte.

9. Un procédé tel que revendiqué dans la revendication 8, dans lequel l'élément isolant creux constitue l'isolement ou gaîne d'un fil électrique et l'électrode intérieure allongée constitue le conducteur du fil électrique.

10. Un procédé tel que revendiqué dans la revendication 9, dans lequel l'élément isolant a été axialement étiré au cours de la fabrication du fil électrique.

11. Un procédé tel que revendiqué dans la revendication 8 ou la revendication 9, qui comporte l'étape qui consiste à étirer l'élément isolant dans la direction axiale avant que l'élément isolant et l'électrode intérieure soient introduits dans l'électrode extérieure.

12. Un procédé tel que revendiqué dans la revendication 8, qui comporte les étapes qui consistent:

(i) à positionner l'élément isolant autour de l'électrode intérieure; puis

(ii) à étirer l'élément isolant dans la direction axiale pour réduire son diamètre extérieur avant que cet élément et l'électrode intérieure soient positionnés à l'intérieur de l'électrode extérieure.

13. Un procédé tel que revendiqué dans l'une qualconque des revendications 8 à 12, dans lequel l'élément isolant est à reprise thermique de ses dimensions et on provoque sa reprise en le chauffant.

# Fig.1.

24
20
22
16
25
18
2     2
14
12
10
30
28   26   32   34

# Fig.2.

14   16   12

*Fig . 3A.*

$L_1$

$d_1$

*12*   *16*

*Fig . 3B.*

$L_2$

$d_2$

*12*   *16*

*Fig . 3C.*

$L_2$

$d_2$   $d_3$

*12*   *14*   *16*

*Fig . 3D.*

$L_3$

*12*   *14*   *16*

2

Fig.4A.

12    16

Fig.4B.

14

12    16

Fig.4C.

12    14    16

Fig.5A.

40    41
14
12
16

Fig.5B.

41
12
41    14
40
16